# EUROPEAN PATENT APPLICATION

(11) **EP 4 725 533 A1**
(43) Date of publication of application: **15.04.2026**
(21) Application number: 25223586.6
(22) Date of filing: 24.09.2015
(51) Int. Cl.: A61M 16/08

(54) **TUBES FOR MEDICAL SYSTEMS**

(30) Priority: 24.09.2014 US 201462054588 P; 28.07.2015 US 201562198038 P
(62) Divisional of application: 22170519.7
(71) Applicant: Fisher & Paykel Healthcare Limited, East Tamaki, Auckland 2013 (NZ)
(72) Inventor: STOKS, Elmo Benson, 2013 Auckland (NZ); MALLINSON, Jayananda, 2013 Auckland (NZ)
(74) Representative: Holt, Lucy Rose

(57) **Abstract**

A medical tube transports gases to and/or from a patient. The medical tube has an elongate film spirally wrapped with an elongate reinforcing member, which bond to form a lumen. The elongate film has a profile such that the elongate film does not protrude into the lumen of the medical tube upon bending of the medical tube. The elongate reinforcing member can have a D-shaped cross section that can contribute to beneficial characteristics of the medical tube. The elongate reinforcing member can also have a circular cross section.

## Description

### PRIORITY

This application claims priority to U.S. Patent Application No. 62/198,038, filed July 28, 2015, and U.S. Patent Application No. 62/054,588, filed September 24, 2014, the entire contents of each of which are incorporated herein by reference.

### BACKGROUND

### Technical Field

The present disclosure generally relates to tubes configured for use in medical systems. More particularly, the present disclosure relates to tubes configured to attach to patient interfaces in respiratory assistance systems.

### Description of the Related Art

Medical tubes are used in respiratory systems to convey respiratory gases between a respiratory component, such as a ventilator or a humidifier, and a patient. Respiratory gases can be heated and/or humidified prior to delivery to the patient to mimic the transformation of air that occurs as it enters the respiratory system. Heated medical tubes can deliver the heated and/or humidified respiratory gases directly to a patient interface or, in some cases, an additional medical tube can be located between the medical tube and the patient interface. The medical tube can be insulated and/or heated to reduce condensate formation within the tube. Breathable tubes can also remove condensate that is formed within the tube. As used herein, the term "breathable" generally means highly permeable to water vapor and substantially impermeable to liquid water and the bulk flow of gases.

### SUMMARY

Although medical tubes exist in the prior art, it is recognised that there are problems with current medical tubes that extend between a respiratory component and a patient interface. Large diameter tubes are often used because decreasing the diameter of a medical tube can result in an increase in resistance to flow. However, some of these larger diameter tubes can also have high resistance to flow. Large medical tubes can also be heavy, bulky, and inflexible in use. They also may not be aesthetically pleasing to a patient, which can result in the treatment not being readily accepted by the patient. Because medical tubes stretch between a respiratory component and a patient interface, they can drag on the patient interface, causing discomfort to the patient and/or disrupting the treatment. Tubes can be noisy when moved or pushed against surfaces, causing the treatment to be obtrusive to the patient and/or a bed partner.

Some medical tubes may not provide good crush resistance. As a result, resistance to flow can be impaired following a crushing event in which the shape and/or lumen of the tube is disrupted. Similarly, the medical tube may not recover from the event but may continue to provide impaired treatment until intervention from the user or may be rendered useless.

A breathable tube can comprise a breathable film reinforced with a more rigid reinforcing member. Current breathable tubes may not be sufficiently robust and, as a result, can be easily damaged in use. When bent, the film may collapse and protrude into the lumen of the tube such that resistance to flow is increased. The breathable film may also be noisy such that it can be disruptive to the patient and/or bed partner.

A medical tube has been developed that at least partially ameliorates or overcomes at least one disadvantage of prior art tubes.

The medical tube can be a smaller diameter tube that provides better flexibility and crush resistance as well as a decreased resistance to flow when compared to existing tubes. The medical tube can extend between a respiratory component and a patient interface. The medical tube can comprise an extruded reinforcing member spirally wound with an extruded film.

A profile of the film can be controlled so that when the medical tube is bent, the film does not collapse into the lumen of the tube. The profile can comprise an inwardly biased profile between adjacent windings of the reinforcing member. The term "inwardly biased," as herein described, generally refers to a configuration in which the film extending between adjacent windings of the reinforcing member drapes toward the center of the lumen when the medical tube is not subject to deformational strain (that is, the medical tube is in a neutral position). The dimensions of the profile can allow the film to drape to a maximal level between adjacent windings of the reinforcing member without protruding into the lumen of the medical tube, thus minimising the effect bending the medical tube may have on resistance to flow.

Flexibility can be improved due to the extent that the film drapes between adjacent windings of the reinforcing member. This can improve the extensibility and bend radius of the medical tube. The pitch of the reinforcing member can also be controlled to improve flexibility. A relationship between the pitch, height, and width of the cross section of the reinforcing member can provide improved tube characteristics.

The shape of the cross section of the reinforcing member can be selected to reduce resistance to flow and to reduce the size of the cavities that exist between adjacent windings of the reinforcing member. The shape can also be important to provide a sufficient bonding surface between the film and the reinforcing member.

The reinforcing member can help to reduce vulnerability of the medical tube to crushing, and the medical tube can recover well following a crushing event or other application of force. In some embodiments the reinforcing member can roll or lean sideways and prevent the medical tube from being crushed, which can improve the recovery of the medical tube from the applied force.

The medical tube can be made to be much smaller in diameter than other medical tubes. In general, the diameter of the medical tube can be in the range of 1 and 20 mm (or thereabout), such as in the range of 7 and 16 mm (or thereabout). For example, small medical tubes, in the range of 14 and 16 mm (or thereabout) in diameter, can be provided to obstructive sleep apnea (OSA) patients, and even smaller medical tubes, in the range of 10 and 12 mm (or thereabout) or in the range of 7 and 9 mm (or thereabout) in diameter, can be provided to high flow therapy (HFT) patients. Smaller medical tubes can improve patient perception of the treatment and can increase patient comfort by providing lighter weight tubes to connect between the respiratory component and the patient interface. Lighter weight and more flexible tubes can also reduce tube drag on the patient interface.

The materials used in the medical tube can also allow quieter and less obtrusive treatment to be provided to patients. As a result, patients may be more accepting of the treatment. Improving patient perception may also improve patient compliance.

In certain embodiments, some or all of the walls surrounding the lumen of the medical tube can comprise a breathable film made of a breathable material. A "breathable film" as herein described refers to a film that is highly permeable to water vapor and substantially impermeable to liquid water and the bulk flow of gases. Similarly, a "breathable material" generally refers to a material that is highly permeable to moisture vapor and substantially impermeable to liquid moisture and the bulk flow of gases. In certain embodiments, a breathable material has a moisture (water) vapor transmission rate greater than or equal to 650 g/m²/day (or thereabout) when measured according to Procedure B of ASTM E96 (using the upright cup method at a temperature of 23 °C and a relative humidity of 50%). The thickness of the breathable film can provide sufficient breathability and flexibility as well as strength and robustness to the medical tube.

A breathable film can advantageously help to expel condensate formed within the medical tube. At least one additional characteristic(s) of a medical tube disclosed herein is or are applicable to a medical tube comprising a film made of a breathable material. It should be understood, however, that at least one characteristic of a medical tube disclosed herein is applicable to a medical tube comprising a film made of a material that is not breathable.

According to at least one aspect of the present disclosure, a first medical tube to transport gases comprises an elongate film spirally wrapped with an elongate reinforcing member to form a lumen. The elongate film bonds with the elongate reinforcing member. The elongate film comprises a profile that keeps the elongate film from protruding into the lumen of the first medical tube when the first medical tube is bent.

The elongate film can be made of a breathable material. The elongate film can be wrapped around a radially-outward surface of the elongate reinforcing member, facing away from the lumen, such that the elongate reinforcing member interacts with the lumen of the first medical tube and the elongate film forms the outer surface of the first medical tube. The longitudinal distance between corresponding points on adjacent windings of the elongate reinforcing member, measured when the first medical tube is not subject to deformational strain, can be selected such that the elongate film drapes a maximal amount between successive windings of the elongate reinforcing member while not extending inwardly beyond the elongate reinforcing member base into the lumen when the first medical tube is bent. The average radial distance from the lowest point of the elongate film in the lumen to the bottom of the elongate reinforcing member, measured when the first medical tube is not subject to deformational strain, can be less than 0.2 mm.

The profile of the first medical tube can be an inwardly biased profile. The elongate reinforcing member can comprise a D-shaped cross section. The flat part of the D-shaped cross section can be longitudinally aligned with the lumen. The semi-circular part of the D-shaped cross section can be facing away from the lumen. The elongate film can bond to the semi-circular part of the D-shaped cross section. Alternatively, the elongate reinforcing member can comprise a circular cross section. Windings of the elongate reinforcing member can roll or lean sideways and prevent the first medical tube from being crushed in response to an applied force. The first medical tube can maintain a gases flow while such a force is applied. A maximal amount of the elongate film can extend between adjacent windings of the elongate reinforcing member without protruding into the lumen. The elongate reinforcing member can be hollow. The elongate reinforcing member can comprise a cavity configured to hold or transport a fluid.

According to at least one aspect of the present disclosure, a second medical tube to transport gases comprises an elongate film spirally wrapped with an elongate reinforcing member to form a lumen. The elongate reinforcing member comprises a D-shaped cross section. The elongate film bonds to a semi-circular part of the D-shaped cross section of the elongate reinforcing member. A maximal amount of the elongate film extends between adjacent windings of the elongate reinforcing member without protruding into the lumen.

The elongate film can be made of a breathable material. The elongate film can be wrapped around a radially-outward surface of the elongate reinforcing member, facing away from the lumen, such that the elongate reinforcing member interacts with the lumen of the second medical tube and the elongate film forms the outer surface of the second medical tube. The average radial distance from the lowest point of the elongate film in the lumen to the bottom of the elongate reinforcing member, measured when the second medical tube is not subject to deformational strain, can be less than 0.2 mm. The profile can be an inwardly biased profile. The flat part of the D-shaped cross section can be longitudinally aligned with the lumen. The semi-circular part of the D-shaped cross section can be facing away from the lumen. Windings of the elongate reinforcing member can roll or lean sideways in response to an applied force. The medical second tube can maintain a gases flow while the force is applied. The elongate reinforcing member can be hollow. The elongate reinforcing member can comprise a cavity configured to hold or transport a fluid.

### BRIEF DESCRIPTION OF THE DRAWINGS

Fig. 1 is a schematic of a respiratory system.
Fig. 2 shows a perspective view of a medical tube according to an embodiment of the disclosed apparatus and systems.
Fig. 3A shows a first cross section of the medical tube of Fig. 2.
Fig. 3B shows the first cross section of the medical tube of Fig. 2 in a bent configuration.
Fig. 3C shows a perspective view of the medical tube of Fig. 2 in a bent configuration.
Fig. 3D shows a perspective, cross-sectional view of the medical tube of Fig. 2.
Fig. 3E shows a plan view of an end of the medical tube of Fig. 3D.
Fig. 3F shows a side plan, cross-sectional view of the medical tube of Fig. 3D.
Fig. 3G shows a detail of the side plan view of Fig. 3F.
Fig. 3H shows a perspective, cross-sectional view of the medical tube of Fig. 3D in a bent configuration.
Fig. 3I shows a detail of the perspective view of Fig. 3H.
Fig. 4A shows a perspective, cross-sectional view of the medical tube of Fig. 2 in a bent configuration.
Fig. 4B shows a detail of the perspective view of Fig. 4A.
Fig. 5A shows a perspective, cross-sectional view of the medical tube of Fig. 2 in a bent configuration.
Fig. 5B shows a side plan, cross-sectional view of the medical tube of Fig. 5A.
Fig. 5C shows a first detail of the side plan view of Fig. 5B.
Fig. 5D shows a second detail of the side plan view of Fig. 5B.
Fig. 6A shows a cross section of an elongate reinforcing member of the medical tube of Fig. 2.
Fig. 6B shows an alternative cross section of an elongate reinforcing member of the medical tube of Fig. 2.
Fig. 6C shows an alternative cross section of an elongate reinforcing member of the medical tube of Fig. 2.
Fig. 7A shows the medical tube of Fig. 2 responding to an applied force.
Fig. 7B shows a side view of the medical tube of Fig. 2.
Fig. 7C shows a plan view of an end of the medical tube of Fig. 7B.
Fig.s 8A-8C show an apparatus for performing flexibility testing.

### DETAILED DESCRIPTION

A respiratory system as herein described can refer to a system that delivers respiratory gases, such as oxygen, carbon dioxide, and/or air to a patient. Any combinations of respiratory gases may also be possible. The patient may be receiving high flow therapy (HFT), treatment for obstructive sleep apnea (OSA), invasive ventilation (INV), or non-invasive ventilation (NIV).

A respiratory component as herein described can refer to, but is not limited to, a gases source, humidification apparatus, humidification chamber, or medical tube.

A medical tube as herein described can refer to a tube, for example, an inspiratory tube, expiratory tube, or interface tube, that connects between a respiratory component and a patient interface.

A patient interface as herein described can refer to, but is not limited to, a mask, oral mask, nasal mask, nasal cannula, nasal pillows, endotracheal tube, or tracheal mask.

A gases source as herein described can refer to an apparatus that supplies gases to a respiratory system such that the gases can be delivered to a patient. The gases source can, for example, take the form of a ventilator, blower, wall source, or gases canister. In some cases, the gases source can be integrated with a humidification apparatus.

"Breathable film" as herein described refers to a film that is highly permeable to moisture vapor such as water vapor, but is substantially impermeable to liquid moisture such as liquid water and substantially impermeable to the bulk flow of gases. Similarly, a "breathable material" generally refers to a material that is highly permeable to moisture vapor and substantially impermeable to liquid moisture and the bulk flow of gases. In certain embodiments, a breathable material has a moisture (water) vapor transmission rate greater than or equal to 650 g/m²/day (or thereabout) when measured according to Procedure B of ASTM E96 (using the upright cup method at a temperature of 23 °C and a relative humidity of 50%).

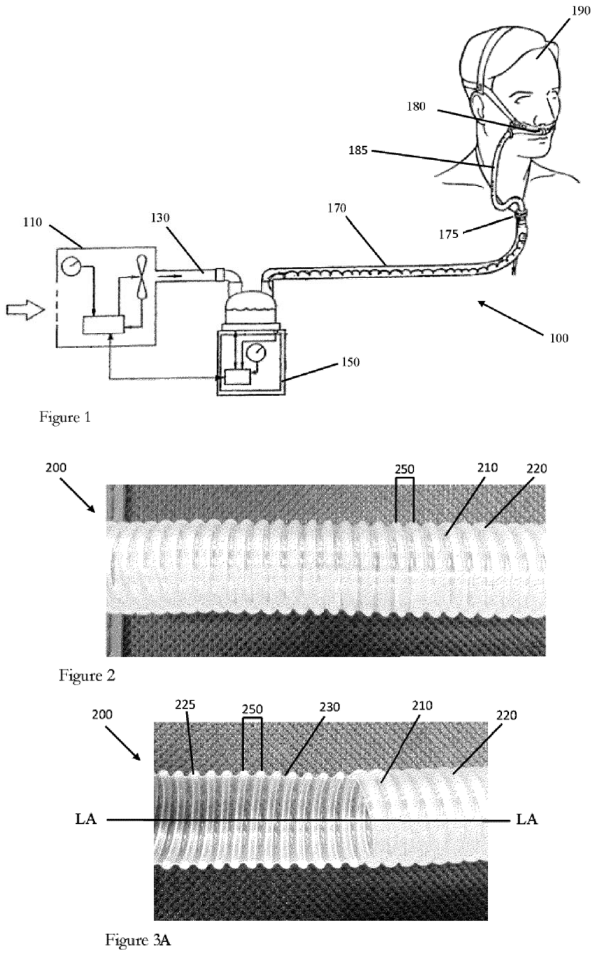

Fig. 1 shows an example of a typical respiratory system 100 having a gases source 110 that is either integrated with, or a separate component from, a humidification apparatus 150. The gases source 110 supplies heated and humidified gases to a patient 190 via a breathing circuit that includes, for example, an inspiratory tube 170 and a patient interface 180. In some embodiments, another medical tube, such as a supply tube 130, can be used to transport gases from the gases source 110 to the humidification apparatus 150. In some embodiments, an additional tube, such as an interface tube 185, can connect between the inspiratory tube 170 and the patient interface 180. In some embodiments, a connector 175 can connect between the inspiratory tube 170 and the interface tube 185. In some embodiments, exhaled gases can be transported via a medical tube, such as an expiratory tube (not shown). In some embodiments that include an expiratory tube, the connector 175 can comprise a wye-piece (not shown) that connects both the inspiratory tube 170 and the expiratory tube to the interface tube 185. It is to be understood that other variations from the system shown may exist.

Fig. 2 shows a medical tube 200 configured to extend between two components of the respiratory system 100. For example, the medical tube 200 can be an embodiment of the interface tube 185, which extends between the connector 175 and the patient interface 180. The medical tube 200 can comprise an elongate film 210 and an elongate reinforcing member 220 that are extruded and spirally wound to form the medical tube 200. In some embodiments, the elongate film 210 can be configured to be breathable such that condensate formed at, for example, the patient interface 180, the connector 175, or the medical tube 200 can be vaporized (e.g., by a heater wire) and transferred through the elongate film 210 to the surrounding atmosphere if the condensate drains back to the medical tube 200. The elongate reinforcing member 220 can provide rigidity and/or structural support to the elongate film 210. In some embodiments, the elongate reinforcing member 220 can comprise at least one wire, which can provide a heating and/or sensing component to the medical tube 200.

The elongate film 210 can be in the range of 50 and 200 µm (or thereabout) thick. In some embodiments, the elongate film 210 can be in the range of 50 and 75 µm (or thereabout) thick. The thickness of the elongate film 210 can be important to reduce or eliminate the likelihood of the medical tube 200 being damaged by the application of reasonable force. Reasonable force refers to a force that the medical tube 200 is expected to encounter during normal use. Forces applied to the medical tube 200 can be directed to the elongate film 210 and, thus, the elongate film 210 can determine the tensile strength of the medical tube 200.

In certain embodiments, the elongate film 210 can have a tensile strength at 100% elongation greater than or equal to 650 lb/in² (psi) (4.5 MPa) (or thereabout) and/or a tensile strength at 300% elongation greater than or equal to 1200 lb/in² (8.3 MPa) (or thereabout). For example, the elongate film 210 can have a tensile strength at 100% elongation equal to 900 lb/in² (6.2 MPa) and a tensile strength at 300% elongation equal to 1700 lb/in² (11.7 MPa) (or thereabout). If the elongate film 210 is too thin it may be more vulnerable to damage, such as punctures or tearing, whereas if the elongate film 210 is too thick it may impair the breathability characteristics and may decrease the flexibility of the elongate film 210. Thus, the thickness of the elongate film 210 can be selected to consider the breathability, flexibility, and robustness of the elongate film 210 as desired for different applications. For example, the elongate film 210 can have a moisture (water) vapor transmission rate greater than or equal to 650 g/m²/day (or thereabout) when measured according to Procedure B of ASTM E96 (using the upright cup method at a temperature of 23 °C and a relative humidity of 50%) and a tensile strength at 100% elongation greater than or equal to 650 lb/in² (4.5 MPa) (or thereabout) and/or a tensile strength at 300% elongation greater than or equal to 1200 lb/in² (8.3 MPa) (or thereabout). Insulating properties of the medical tube 200 can also be important in reducing the amount of condensate within the medical tube 200.

Fig. 3A shows a cross section of the medical tube 200. Each of the elongate film 210 and the elongate reinforcing member 220 are spirally wound to form the medical tube 200 having a longitudinal axis LA-LA and a lumen (tube bore) extending along the longitudinal axis LA-LA. It can be seen that, in some embodiments, the elongate film 210 can have a profile 230. The profile 230 can be shaped such that it is inwardly biased toward the lumen of the medical tube 200, which can improve the performance of the medical tube 200, especially when compared with other medical tubes.

In some embodiments, the elongate film 210 can be made from a breathable thermoplastic material, such as a thermoplastic elastomer (or TPE as defined by ISO 18064:2003(E)), a thermoplastic polyurethane (or TPU as defined by ISO 18064:2003(E)), a thermoplastic polyester, or other material with elastomeric properties. The elongate reinforcing member 220 can be made from, for example, a TPU. The materials disclosed are not meant to be limiting but rather are examples of possible materials that can be used. The materials can be chosen such that a bond is formed between the elongate film 210 and the elongate reinforcing member 220. The materials can be chosen such that when the medical tube 200 moves and/or contacts other surfaces, it remains quiet and unobtrusive. Different materials and/or material combinations can fall within the scope of this disclosure.

The elongate film 210 can be wrapped around the outside of the elongate reinforcing member 220 such that the elongate reinforcing member 220 interacts with the lumen of the medical tube 200 and the elongate film 210 forms the outer surface of the medical tube 200. In some embodiments, wherein the elongate film 210 comprises a breathable material, this can allow more of the breathable surface of the elongate film 210 to be exposed to the ambient environment such that a greater amount of moisture can be lost from the elongate film 210. Thus, the inwardly biased profile 230 of the medical tube 200 can reduce substantially greater amounts of condensate formed when compared with other medical tubes. The cavities between the elongate film 210 and the elongate reinforcing member 220 can be small when the medical tube 200 has the inwardly biased profile 230, which can result in a reduced resistance to flow in the lumen of the medical tube 200.

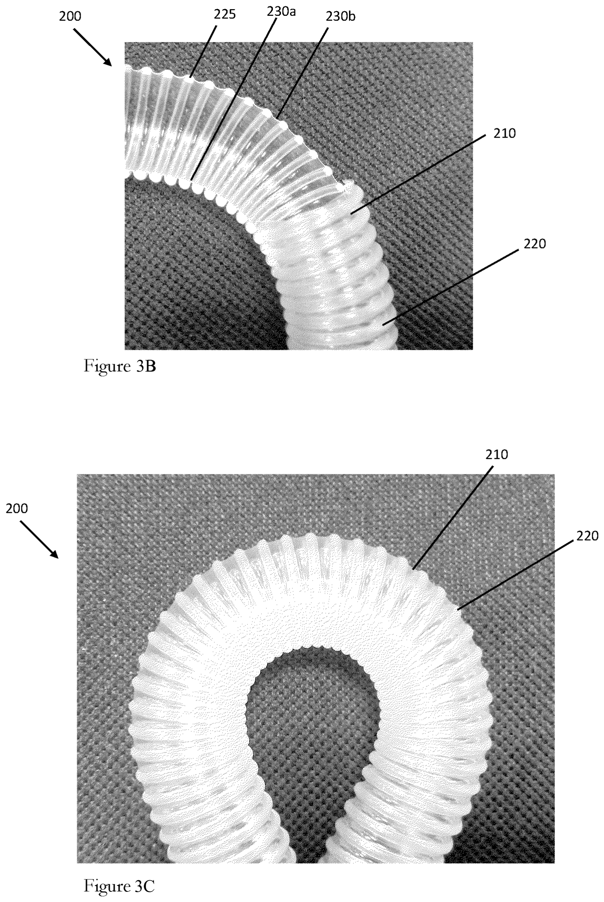

Figs. 3B and 3C illustrate a bent configuration of the medical tube 200. The elongate film 210 on the outer side 230b of the bend can flatten slightly while the elongate film 210 on the inner side 230a of the bend can collapse inwardly toward the lumen of the medical tube 200. A maximal amount of the elongate film 210 between adjacent windings of the elongate reinforcing member 220 can allow for greater flexibility, extension, and/or an improved bend radius of the medical tube 200. Thus, the medical tube 200 can be able to bend around a small bend radius without the elongate film 210 protruding into the lumen of the medical tube 200. As a result, the medical tube 200 can be bent sharply without substantially impacting resistance to flow.

Reducing the resistance to flow in the lumen of the medical tube 200 can allow the diameter of the medical tube 200 to be smaller than that of other medical tubes while maintaining a similar or improved performance level. In some embodiments, the diameter of the medical tube 200 can be in the range of 10 and 12 mm (or thereabout) or in the range of 7 and 9 mm (or thereabout) for HFT patients, and in the range of 14 and 16 mm (or thereabout) for OSA patients. In some embodiments, the diameter of the medical tube 200 can be as small as in the range of 1 and 2 mm (or thereabout), or as large as may be achieved given any limitations of the manufacturing processes employed.

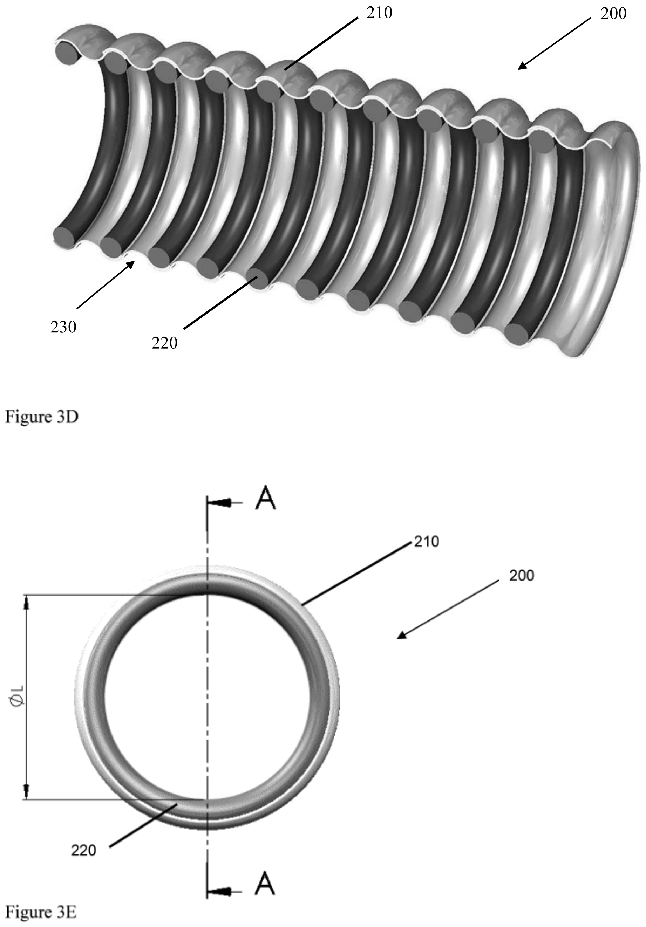

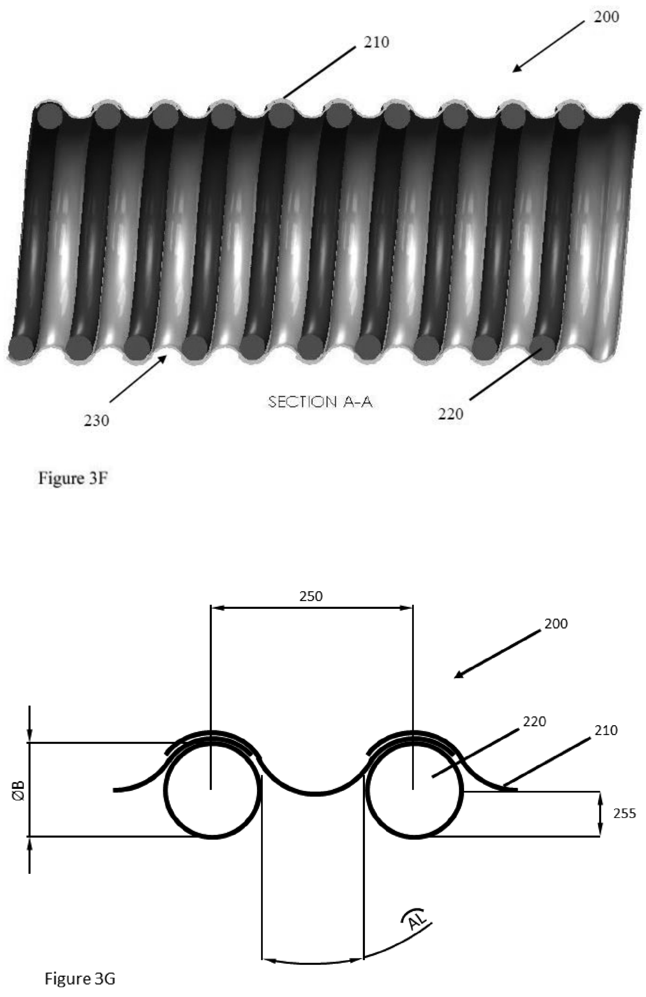

Fig. 3D shows a perspective, cross-sectional view of the medical tube 200 of Fig. 2. Fig. 3E shows a plan view of an end of the medical tube 200 in Fig. 3D. Fig. 3F shows a side plan, cross-sectional view of the medical tube 200, taken along section line A-A shown in Fig. 3D. Fig. 3G shows a detail of the side plan view of Fig. 3F.

The inwardly biased profile 230 of the elongate film 210 is shown in Figs. 3D and 3F. As discussed above with reference to Figs. 3B and 3C, the amount of the elongate film 210 between adjacent windings of the elongate reinforcing member 220 provides sufficient flexibility of the medical tube 200 without substantially impacting resistance to flow. In other words, the medical tube 200 is flexible, but the elongate film 210 does not protrude into the lumen of the medical tube 200. The diameter ØL of the lumen is shown in the plan view of Fig. 3E.

The dimensions of the medical tube 200 are interrelated and affect a satisfactory design. For example, a relationship can exist between the amount of the elongate film 210 extending between adjacent windings of the elongate reinforcing member 220 and a pitch 250 of the elongate reinforcing member 220, to determine the extent to which the elongate film 210 drapes toward the lumen between adjacent windings of the elongate reinforcing member 220. Pitch as herein described refers to a longitudinal distance between corresponding points on adjacent windings of the elongate reinforcing member 220 measured when the medical tube 200 is not subject to deformational strains. For example, the pitch 250 can be measured as the longitudinal distance between the centers of two adjacent windings of the elongate reinforcing member 220, as shown in Figs. 3A and 3G.

A minimum amount of the elongate film 210 required to extend between adjacent windings of the elongate reinforcing member 220, for a given magnitude of the pitch 250, can be estimated by subtracting the width of the elongate reinforcing member 220 from the pitch 250 of the elongate reinforcing member 220. In addition, as shown in Fig. 3G, the pitch 250 and the amount of the elongate film 210 extending between adjacent windings of the elongate reinforcing member 220 can be selected to give an arc length AL for the elongate film 210 that does not protrude into the lumen of the medical tube 200.

The pitch 250 is an important feature. If the pitch 250 is too small, the flexibility of the medical tube 200 increases, but the bend radius of the medical tube 200 is negatively affected due to bunching of adjacent windings of the elongate reinforcing member 220. Desirably, the pitch 250 is selected to maintain a bend radius greater than or equal to 10 mm (or thereabout). If the pitch 250 is too large, the elongate film 210 can protrude too far into the lumen of the medical tube 200, and the medical tube 200 can be more expensive due to the increased cost of the elongate film 210.

To improve the desired characteristics of the medical tube 200, the amount of the elongate film 210 extending between adjacent windings of the elongate reinforcing member 220 and/or the pitch 250 can be selected such that the profile 230 allows the elongate film 210 to drape as much as possible between adjacent windings of the elongate reinforcing member 220, while not protruding inwardly beyond the elongate reinforcing member 220 into the lumen of the medical tube 200 when the medical tube 200 is bent. As a result, when the medical tube 200 is bent as shown in Figs. 3B and 3C, the elongate film 210 at the inner side 230a is less likely to protrude into the lumen of the medical tube 200. This can favorably affect resistance to flow of the medical tube 200 when compared with other medical tubes.

As discussed above, a bond can be formed between the elongate film 210 and the elongate reinforcing member 220. To further improve the desired characteristics of the medical tube 200, the dead space between the elongate film 210 and the elongate reinforcing member 220 can be selectively controlled. Dead space as herein described refers to a cross sectional area between corresponding points on adjacent windings of the elongate reinforcing member 220 and under the elongate film 210 and above the lowest point of the elongate reinforcing member 220 within the lumen, measured when the medical tube 200 is not subject to deformational strains. With reference to Fig 3G, for example, the dead space represents the cross sectional area within the lumen that is above the lower line of ØB, below the elongate film 210, and between the lines indicating the pitch 250. An example metric reflecting dead space is the inward bias dimension, defined herein as an average radial distance 255 between the lowest point of the elongate film 210 in the lumen and the bottom of the elongate reinforcing member 200 between corresponding points on adjacent windings of the elongate reinforcing member 200. With reference again to Fig. 3G, the inward bias dimension represents the average radial distance 255 between the lowest point of the elongate film 210 between the lines of the pitch 250 and the lower line of ØB.

In a desirable configuration, an average inward bias dimension is less than 0.2 mm (or thereabout) in the medical tube 200 having an average of the pitch 250 of 2 mm (or thereabout), an average of the inner diameter ØL of 7.5 mm (or thereabout), an average of the diameter ØB of the elongate reinforcing member 220 of 0.8 mm (or thereabout), and an average thickness of the elongate film 210 of 0.08 mm (or thereabout). For example, an acceptable configuration can have an average inward bias dimension of 0.06 mm in the medical tube 200 having an average of the pitch 250 of 1.71 mm, an average of the inner diameter ØL of 7.6 mm, an average of the diameter ØB of the elongate reinforcing member 220 of 0.78 mm, and an average thickness of the elongate film 210 of 0.08 mm. Another acceptable configuration can have an average inward bias dimension of 0.15 mm in the medical tube 200 having an average of the pitch 250 of 1.71 mm, an average of the inner diameter ØL of 9.52 mm, an average of the diameter ØB of the elongate reinforcing member 200 of 0.86 mm, and an average thickness of the elongate film 210 of 0.08 mm. An example configuration of excessive inward bias dimension can have an average inward bias of 0.2 mm in the medical tube 200 having an average of the pitch 250 of 2.05 mm, an average of the inner diameter ØL of 7.5 mm, an average of the diameter ØB of the elongate reinforcing member 220 of 0.82 mm, and an average thickness of the elongate film 210 of 0.08 mm. Another example configuration of excessive inward bias dimension can have an average inward bias of 0.58 mm in the medical tube 200 having an average of the pitch 250 of 1.95 mm, an average of the inner diameter ØL of 8.43 mm, an average of the diameter ØB of the elongate reinforcing member 220 of 0.9 mm, and an average thickness of the elongate film 210 of 0.15 mm. It should be understood that the scope of this disclosure does not exclude the last two example configurations, even though those configurations include characteristics that can be considered less advantageous under certain circumstances.

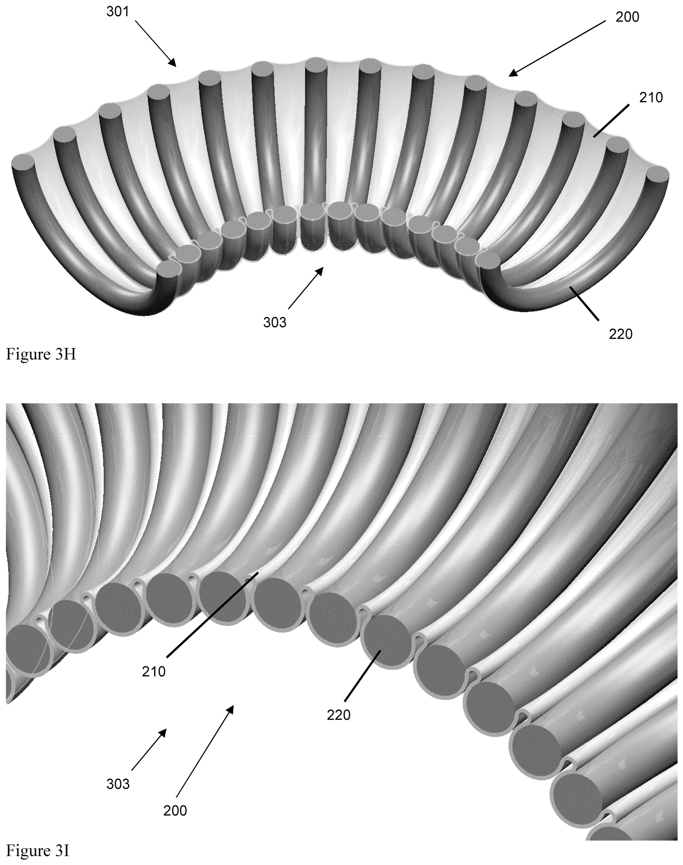

A desirable configuration is further shown in Figs. 3H and 3I. In the top profile 301 of the medical tube 200 of Fig. 3H, the pitch of the elongate reinforcing member 220, combined with the amount of the elongate film 210 extending between adjacent windings of the elongate reinforcing member 220, allows the elongate film 210 to stretch and increases the bend radius of the medical tube 200. In the bottom profile 303 of Fig. 3H, and as shown more particularly in Fig. 3I, the amount of the elongate film 210 extending between adjacent windings of the elongate reinforcing member 220 is small enough that, when the medical tube 200 is bent, the elongate film 210 minimally protrudes into the lumen of the medical tube 200. This is an example of a successful tube design that can account for increasing bend radius while reducing resistance to flow.

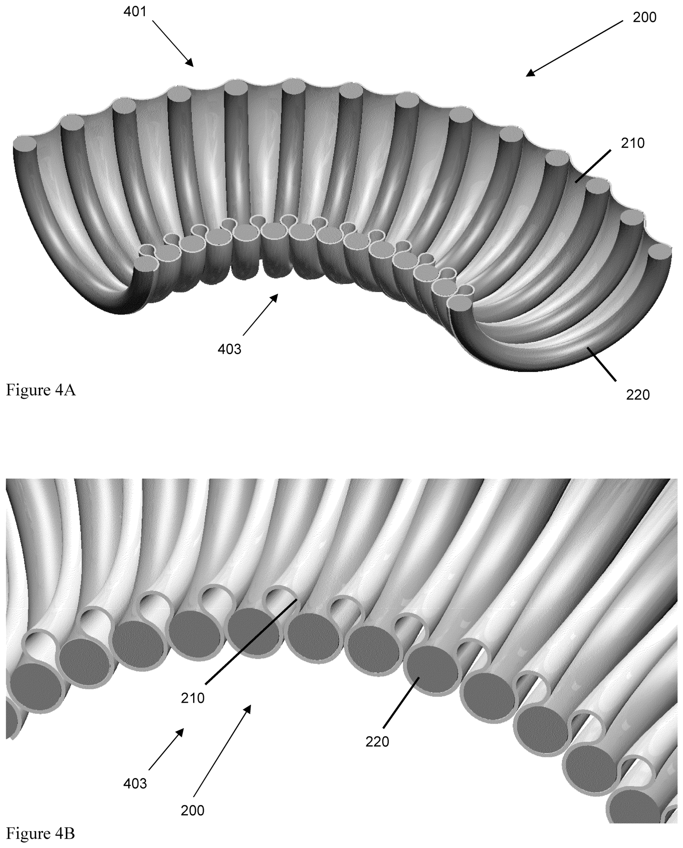

Fig. 4A shows a perspective, cross-sectional view of the medical tube 200 of Fig. 2 in a bent configuration. Fig. 4B shows a detail of the perspective view of Fig. 4A. This embodiment of the medical tube 200 does not successfully account for increasing bend radius while reducing resistance to flow. In this embodiment of the medical tube 200, the pitch of the elongate reinforcing member 220, combined with the amount of the elongate film 210 extending between adjacent windings of the elongate reinforcing member 220, does desirably improve bend radius. However, in the top profile 401 of the medical tube 200 of Fig. 4A, the excessive amount of the elongate film 210 extending between adjacent windings of the elongate reinforcing member 220 causes the elongate film 210 to drape and protrude into the lumen of the medical tube 200 when the medical tube 200 is bent, which increases resistance to flow by reducing the effective bore size. In the bottom profile 403 of Fig. 4A, and as shown more particularly in Fig. 4B, the excessive amount of the elongate film 210 extending between adjacent windings of the elongate reinforcing member 220 causes the elongate film 210 to significantly protrude into the lumen of the medical tube 200 between adjacent windings of the elongate reinforcing member 220 when the medical tube 200 is bent, which further increases resistance to flow. It should be understood that the scope of this disclosure does not exclude the embodiment of generalized Fig. 4, even though that embodiment includes characteristics that can be considered less advantageous under certain circumstances.

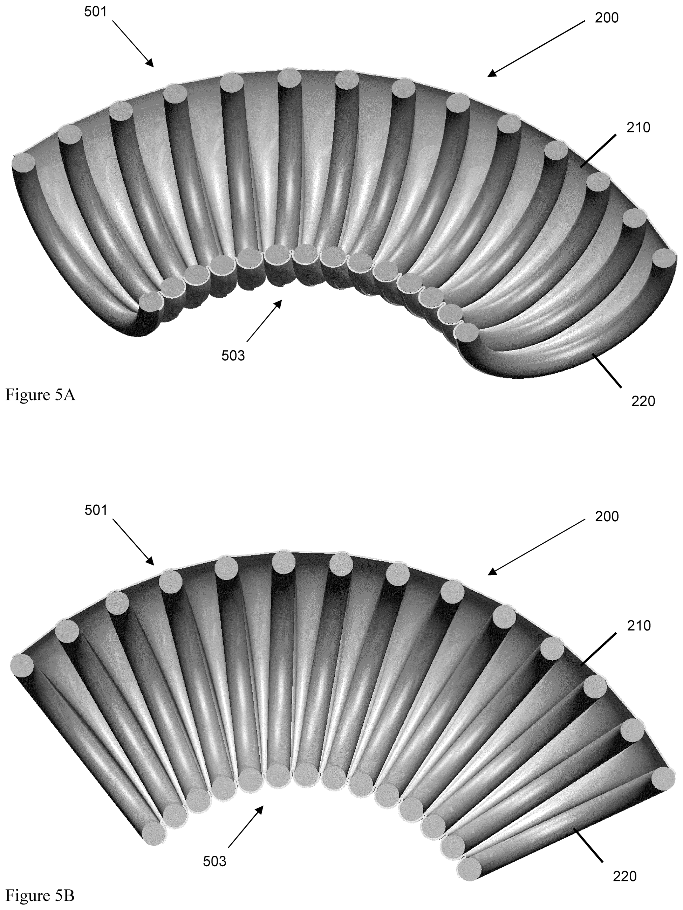

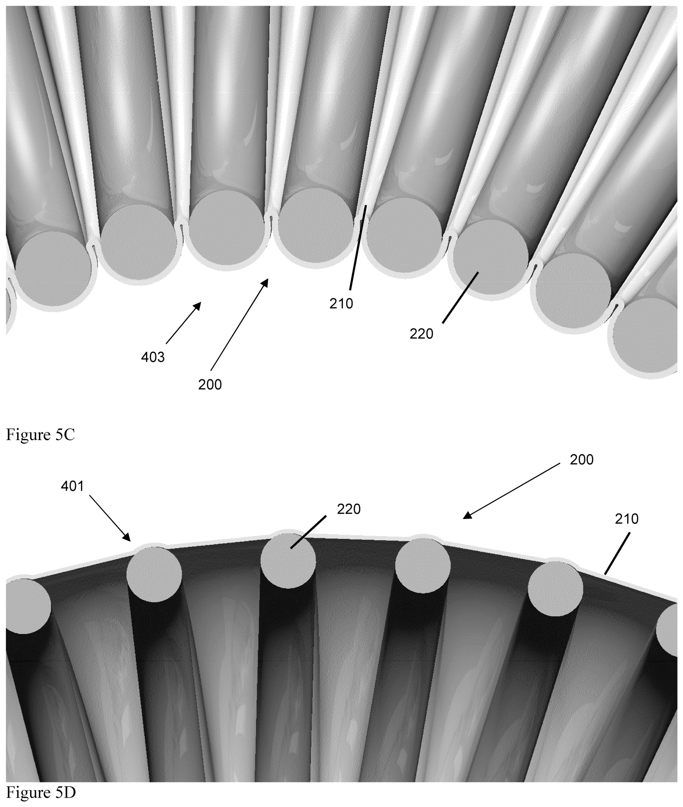

Fig. 5A shows a perspective, cross-sectional view of the medical tube 200 of Fig. 2 in a bent configuration. Fig. 5B shows a side plan, cross-sectional view of the medical tube 200 of Fig. 5A. Fig. 5C shows a first detail of the side plan view of Fig. 5B. Fig. 5D shows a second detail of the side plan view of Fig. 5B. This embodiment of the medical tube 200 also does not successfully account for increasing bend radius while reducing resistance to flow. In this embodiment of the medical tube 200, the pitch of the elongate reinforcing member 220, combined with the amount of the elongate film 210 between adjacent windings of the elongate reinforcing member 220, fails to improve bend radius. In the top profile 501 of the medical tube 200 of Fig. 5A and 5B, and as shown more particularly in Fig. 5D, the small amount of the elongate film 210 extending between adjacent windings of the elongate reinforcing member 220 causes the elongate film 210 to pull tight before the medical tube 200 is completely bent. Although the profile 501 does not negatively impact resistance to flow, it negatively impacts bend radius and flexibility. In the bottom profile 503 of Fig. 5A and 5B, and as shown more particularly in Fig. 5C, the small amount of the elongate film 210 extending between adjacent windings of the elongate reinforcing member 220 reduces flexibility and may negatively impact other characteristics. It should be understood that the scope of this disclosure does not exclude the embodiment of generalized Fig. 5, even though that embodiment includes aspects that may be considered less advantageous under certain circumstances.

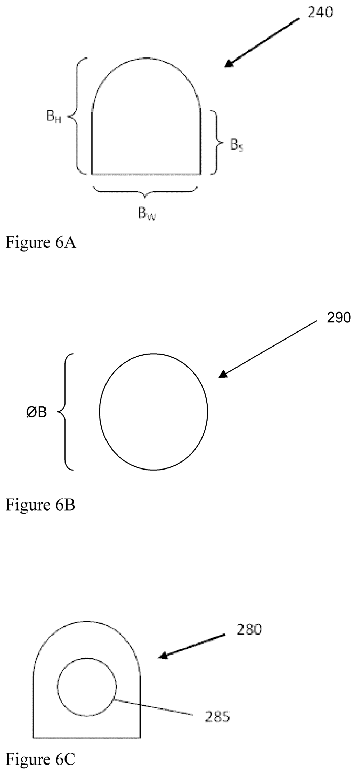

In some embodiments, the shape of the elongate reinforcing member 220 can be selected to further improve the performance of the medical tube 200. Fig. 6A shows, for example, that the cross section of the elongate reinforcing member 220 can have a D-shape 240. In some embodiments, the D-shape 240 can comprise two substantially parallel sides (the height of which is indicated by B_{S}) and a base that connects the two substantially parallel sides (the width of which is indicated by B_{W}). The top of the D-shape 240 can comprise a semi-circular part and the overall height of the elongate reinforcing member 220 is indicated by B_{H}. In some embodiments, different shapes can be used for the cross section of the elongate reinforcing member 220, for example, a square, semi-circular or circular shape, which can comprise substantially parallel and/or non-parallel regions. In some embodiments, the base of the elongate reinforcing member 220 can be substantially flat such that any interaction with the lumen of the medical tube 200 forms minimized cavities between each winding of the elongate reinforcing member 220 and adjacent windings of the elongate film 210. Minimizing the size of the cavities can reduce the resistance to flow of the medical tube 200 when compared with other medical tubes.

The amount of the elongate film 210 extending between adjacent windings of the elongate reinforcing member 220 for a certain diameter of the medical tube 200 can be determined by a relationship that exists between the width of the elongate reinforcing member (B_{W}), the height of the elongate reinforcing member (B_{H}), and the height of each side of the elongate reinforcing member 220 (B_{S}), wherein the height of each side of the elongate reinforcing member 220 can be defined as the length of the portion of that side that is straight. In a non-limiting example, as shown in Fig. 6A, the height of either side of the elongate reinforcing member 220 (B_{S}) can be half of the height of the elongate reinforcing member (B_{H}); however, in some embodiments, the height of either side of the elongate reinforcing member 220 (B_{S}) can change depending on the shape of the elongate reinforcing member 220.

A relationship can also exist between the height of the elongate reinforcing member 220 (B_{H}), the width of the elongate reinforcing member (B_{W}), and the pitch 250 of the elongate reinforcing member 220. As a result, the profile 230 can be selected for the height, the width, and the pitch 250 of the elongate reinforcing member 220. For example, a height for the elongate reinforcing member 220 that is greater than a threshold value may be less stable during manufacturing and may reduce the smoothness of the outer surface of the medical tube 200. The extent to which the elongate film 210 drapes between adjacent windings of the elongate reinforcing member 220 may need to be less than the height of the elongate reinforcing member 220, or the elongate film 210 may need to be bonded to the elongate reinforcing member 220 such that it does not protrude into the lumen of the medical tube 200 when bent. In some embodiments, a solution can be provided taking into account the amount of the elongate film 210 extending between adjacent windings of the elongate reinforcing member 220 and the bonding between the elongate film 210 and the elongate reinforcing member 220.

The elongate reinforcing member 220 can have a substantially flat base to maintain a smoother internal surface of the medical tube 200, expecially when the medical tube 200 is bent. This can contribute to a reduced resistance to flow through the lumen of the medical tube 200. The semi-circular part can give the outside of the medical tube 200 a softer and/or smoother overall feel. The elongate film 210 being on the outer surface of the elongate reinforcing member 220 and, thus, surrounding the elongate reinforcing member 220, can also provide the medical tube 200 with a softer overall feel for the user.

The D-shape 240 can provide a bonding region for the elongate film 210. For example, the elongate film 210 can bond along the curved region defined by the semi-circular part of the D-shape 240. This can improve the flexibility and tensile strength of the medical tube 200 by allowing the elongate film 210 to extend to a maximal length between adjacent windings of the elongate reinforcing member 220. The bonding can also reduce the susceptibility of the medical tube 200 to manufacturing variation, which can make the medical tube 200 more resilient. In some embodiments, the elongate film 210 can bond over a smaller region of the D-shape 240 or can bond over a larger region of the D-shape 240.

The D-shape 240 can improve the crush resistance and/or recovery of the medical tube 200. The top of the elongate reinforcing member 220 comprises a semi-circular part that can provide an unstable surface, which can be better at absorbing or diverting crush forces. Thus, during the application of reasonable force, adjacent windings of the elongate reinforcing member 220 can roll or lean sideways rather than collapsing downward in a way that may allow crushing of the medical tube 200, which can reduce restriction of the lumen of the medical tube 200 and ensure that some flow can be maintained through the medical tube 200. Once the force ceases and/or is removed, the medical tube 200 can recover well and quickly. Thus, the medical tube 200 can regain its original shape with little or no impact on the medical tube 200. The rolling or leaning feature of the elongate reinforcing member 220 can depend on the pitch 250.

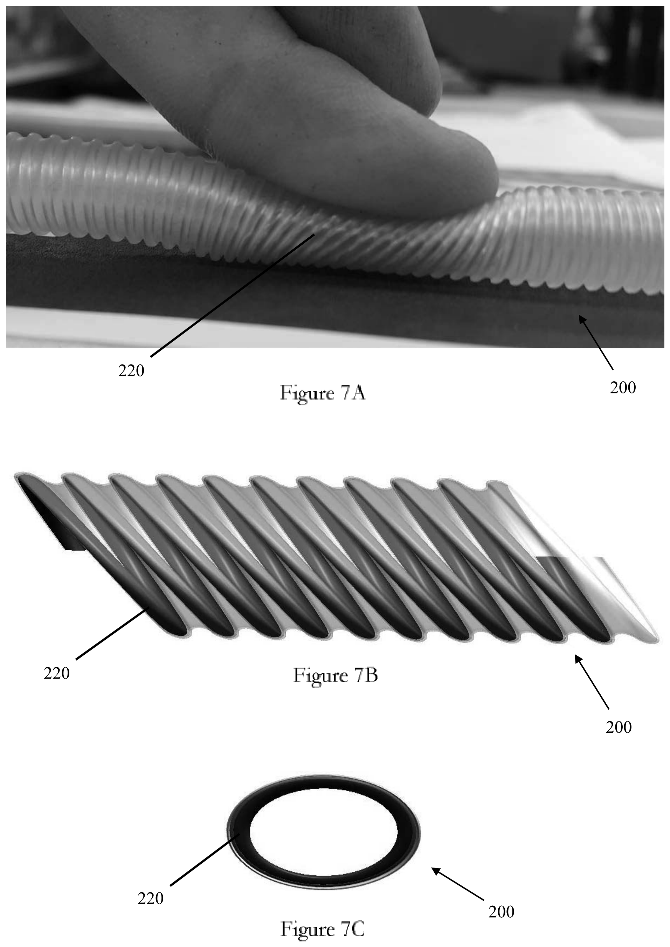

Fig. 7A shows the medical tube 200 responding to the application of reasonable force and illustrates how adjacent windings of the elongate reinforcing member 220 roll or lean sideways rather than collapse downward in a way that can allow crushing of the medical tube 200. It can be seen in Fig. 7A that windings of the elongate reinforcing member 220 also partially compress in response to the application of reasonable force, but not so much as to significantly restrict flow through the medical tube 200. Fig. 7B shows a side view of the medical tube 200 with the elongate reinforcing member 220 rolling or leaning sideways as would occur in response to the application of reasonable force. Fig. 7C shows a plan view of an end of the medical tube 200 with the elongate reinforcing member 220 partially compressed as would occur in response to the application of reasonable force.

In certain embodiments, the elongate reinforcing member 220 has a cross-sectional shape 290 that is substantially circular, as shown in Figs. 3G and 6B. In such embodiments, the circular shape 290 is similar to the D-shape 240 described with reference to Fig. 6A, except that the sharp edges of the D-shape 240 are substantially rounded in the circular shape 290. In other words, the general size (e.g., the diameter ØB) of the circular shape 290 is approximately the same as the general size (e.g., the width B_{W}) of the D-shape 240. It was discovered that substituting the D-shape 240 of the elongate reinforcing member 220 with the circular shape 290 does not substantially impact tube performance. Resistance to flow is only slightly increased for the medical tube 200 when the elongate reinforcing member comprises the circular shape 290, as compared to the D-shape 240. Also, the circular shape 290 can be easier to manufacture than the D-shape 240 in some circumstances. It should be understood that the elongate reinforcing member 220 can comprise other cross-sectional shapes than the D-shape 240 or the circular shape 290, and Figs. 6A and 6B are not limiting.

In some embodiments, the medical tube 200 can comprise a hollow elongate reinforcing member 280, as shown in Fig. 6C. The hollow elongate reinforcing member 280 can be more lightweight and can use less material, as compared to the elongate reinforcing member 220, such that the medical tube 200 with the hollow elongate reinforcing member 280 can be made at a reduced cost. In some embodiments, the hollow elongate reinforcing member 280 comprises a cavity 285 that can be configured to hold or transport additional fluids. For example, the hollow elongate reinforcing member 280 can be configured to transport gases and/or liquids. In one example, the hollow elongate reinforcing member 280 can be configured as a pressure sample line for conveying pressure feedback from the patient end of the medical tube 200 to the humidification apparatus 150. In some embodiments, the hollow elongate reinforcing member 280 can be configured to transport or deliver medicaments. In some embodiments, a temperature of gases transported in the lumen of the medical tube 200 can be determined by measuring a property of a fluid held within the hollow elongate reinforcing member 280. In the example of Fig. 6C, the outer shape of the hollow elongate reinforcing member 280 is substantially the same as the D-shape 240. Nevertheless, it should be understood that the hollow elongate reinforcing member 280 can be implemented with other outer shapes, such as (but not limited to) the substantially circular shape 290 of Fig. 6B.

Examples of the medical tube 200 can be described as follows. These examples are meant to be illustrative only and are in no way limiting, and modifications or variations to the examples given can also fall within the scope of the present disclosure. In general, the diameter of the medical tube 200 can be in the range of 1 and 20 mm (or thereabout), such as in the range of 7 and 16 mm (or thereabout).

In some embodiments, such as embodiments relating to tubes for OSA patients, the diameter of the medical tube 200 can be 15 mm ± 10% and the pitch 250 of the elongate reinforcing member 220 of the medical tube 200 can be 2.0 mm ± 10% or 2.5 mm ± 10%, as measured between adjacent bead windings (as shown in Fig. 2). The bead height (B_{H}) can be 1.3 mm ± 10%, and the bead width (B_{W}) can be 1.3 mm ± 10%. The thickness of the elongate film 210 can be 75 µm ± 10%.

In some embodiments, including some embodiments relating to tubes for HFT patients, the diameter of the medical tube 200 can be in the range of 10 and 12 mm ± 10%, for example 11.8 mm, and the pitch 250 of the elongate reinforcing member 220 of the medical tube 200 can be in the range of 1.8 and 3.0 mm ± 10% or in the range of 1.8 and 3.5 mm ± 10%. The bead height (B_{H}) can be in the range of 0.8 and 1.5 mm ± 10%, and the bead width (B_{W}) can be in the range of 0.8 and 1.5 mm ± 10%. The thickness of the elongate film 210 in this embodiment can be 100 µm ± 10%. In some embodiments, including more embodiments relating to tubes for HFT patients, the diameter of the medical tube 200 can be in the range of 7 and 9 mm ± 10%, for example 8.5 mm, and the pitch 250 of the elongate reinforcing member 220 of the medical tube 200 can be in the range of 2.1 and 2.2 mm ± 10% or in the range of 2.2 and 2.3 mm ± 10%. The bead height (B_{H}) can be 1 mm ± 10%, and the bead width (B_{W}) can be 1 mm ± 10%. The thickness of the elongate film 210 in this embodiment can be 100 µm ± 10%.

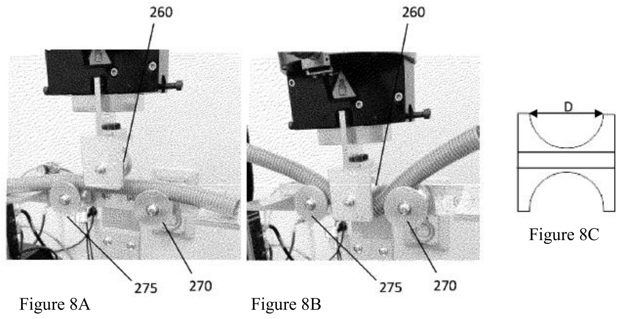

The flexibility of the medical tube 200 can be characterized by the result of a three point bend test as shown in Figs. 8A-8C, wherein the force required to lower a top roller 260 onto a 300 mm section of the medical tube 200 until it has been lowered 50 mm can be between 0.008 and 0.02 N/mm. For the purposes of this test, a first bottom roller 270 and a second bottom roller 275 can be 100 mm apart.

The medical tube 200 can be configured for use on infants, adults, or patients using different therapies, such as NIV, INV, HFT, or OSA patients. Thus, in some embodiments, the respiratory system 100 can comprise the medical tube 200 as well as an expiratory circuit. In this case, the medical tube 200 can connect to a wye-piece or other respiratory component. The tube can be configured to extend between a respiratory component and the patient interface 180 and, in some embodiments, between the inspiratory tube 170 and the patient interface 180. The medical tube 200 can be configured to be directly connected between a respiratory component and the patient interface 180.

Another advantage of the medical tube 200 having a smaller diameter than other medical tubes is that the medical tube 200 can be small, lightweight, and unobtrusive to the patient 190. Improved flexibility and extensibility of the medical tube 200, combined with a lighter weight, makes the medical tube 200 less likely to drag on the patient interface 180 and, thus, more comfortable to the patient 190. As a result, the patient 190 can be more accepting of the treatment and patients can have improved patient compliance.

Various changes and modifications to the presently preferred embodiments described herein will be apparent to those skilled in the art. For example, the diameter of the medical tube 200 and/or the pitch 250 of the elongate reinforcing member 220 of the medical tube 200 can vary for different applications and/or use. Similarly, the shape of the elongate reinforcing member 220 can be selected for different applications where applicable and is in no way limited to the D-shape 240 or the circular shape 290. In some embodiments, the elongate film 210 can comprise a material that is not a breathable material. Such changes and modifications can be made without departing from the spirit and scope of the apparatus and systems of the disclosure and without diminishing its attendant advantages. For instance, various components can be repositioned as desired. It is therefore intended that such changes and modifications be included within the scope of the apparatus and systems of the disclosure.

Moreover, not all of the features, aspects and advantages are necessarily required to practice the present apparatus and systems of the disclosure. Accordingly, the scope of the present apparatus and systems of the disclosure is intended to be defined only by the claims that follow.

Reference to any prior art in this specification is not, and should not be taken as, an acknowledgement or any form of suggestion that that prior art forms part of the common general knowledge in the field of endeavour in any country in the world.

Wherein the foregoing description reference has been made to integers or components having known equivalents thereof, those integers are herein incorporated as if individually set forth.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

The apparatus and system of the disclosure can also be said broadly to consist in the parts, elements and features referred to or indicated in the specification of the application, individually or collectively, in any or all combinations of two or more of said parts, elements or features.

It will be appreciated that there is thus disclosed:
According to the present invention here is provided a medical tube to transport gases, the medical tube comprising: an elongate film spirally wrapped with an elongate reinforcing member to form a lumen, the elongate film bonding with the elongate reinforcing member, wherein the elongate film comprises a profile that keeps the elongate film from protruding into the lumen of the medical tube when the medical tube is bent.

Preferably the elongate film is made of a breathable material.

Preferably the elongate film is wrapped around a radially-outward surface of the elongate reinforcing member, facing away from the lumen, such that the elongate reinforcing member interacts with the lumen of the medical tube and the elongate film forms the outer surface of the medical tube.

Preferably the longitudinal distance between corresponding points on adjacent windings of the elongate reinforcing member, measured when the medical tube is not subject to deformational strain, is selected such that the elongate film drapes a maximal amount between successive windings of the elongate reinforcing member while not extending inwardly beyond the elongate reinforcing member base into the lumen when the medical tube is bent.

Preferably the average radial distance from the lowest point of the elongate film in the lumen to the bottom of the elongate reinforcing member, measured when the medical tube is not subject to deformational strain, is less than 0.2 mm.

Preferably the profile is an inwardly biased profile.

Preferably the elongate reinforcing member comprises a D-shaped cross section.

Preferably the flat part of the D-shaped cross section is longitudinally aligned with the lumen and the semi-circular part of the D-shaped cross section is facing away from the lumen.

Preferably the elongate film bonds to the semi-circular part of the D-shaped cross section. Preferably the elongate reinforcing member comprises a circular cross section.

Preferably windings of the elongate reinforcing member roll or lean sideways in response to an applied force.

Preferably the medical tube maintains a gases flow while the force is applied.

Preferably a maximal amount of the elongate film extends between adjacent windings of the elongate reinforcing member without protruding into the lumen.

Preferably the elongate reinforcing member is hollow.

Preferably the elongate reinforcing member comprises a cavity configured to hold or transport a fluid.

According to the present invention here is provided a medical tube to transport gases, the medical tube comprising: an elongate film spirally wrapped with a elongate reinforcing member to form a lumen, the elongate reinforcing member comprising a D-shaped cross section, the elongate film bonding to a semi-circular part of the D-shaped cross section of the elongate reinforcing member, wherein a maximal amount of the elongate film extends between adjacent windings of the elongate reinforcing member without protruding into the lumen.

Preferably the elongate film is made of a breathable material.

Preferably the elongate film is wrapped around a radially-outward surface of the elongate reinforcing member, facing away from the lumen, such that the elongate reinforcing member interacts with the lumen of the medical tube and the elongate film forms the outer surface of the medical tube.

Preferably the average radial distance from the lowest point of the elongate film in the lumen to the bottom of the elongate reinforcing member, measured when the medical tube is not subject to deformational strain, is less than 0.2 mm.

Preferably the profile is an inwardly biased profile.

Preferably the flat part of the D-shaped cross section is longitudinally aligned with the lumen and the semi-circular part of the D-shaped cross section is facing away from the lumen.

Preferably windings of the elongate reinforcing member roll or lean sideways in response to an applied force.

Preferably the medical tube maintains a gases flow while the force is applied.

Preferably the elongate reinforcing member is hollow.

Preferably the elongate reinforcing member comprises a cavity configured to hold or transport a fluid.

According to the present invention there is provided a medical tube to transport gases, the medical tube comprising an elongate film spirally wrapped with an elongate reinforcing member to form a lumen, the elongate film bonding with the elongate reinforcing member, wherein the elongate film comprises a profile that keeps the elongate film from protruding into the lumen of the medical tube when the medical tube is bent, wherein the profile comprises an inwardly biased profile between adjacent windings of the reinforcing member; wherein the elongate film comprises a breathable material, wherein the elongate reinforcing member is more rigid than the elongate film.

Preferably a thickness of the elongate film is in the range of 50 and 200 µm, preferably 100 µm±10%, and/or wherein the elongate film is made of a polyurethane material, preferably a thermoplastic polyurethane material, and/or wherein the elongate film is made of a material having a vapour transmission rate greater than or equal to 650 g/m²/day.

Preferably the reinforcing member is made of a thermoplastic polyurethane, and/or wherein the diameter of the medical tube is between 1 mm and 16 mm, preferably in the range of 10 and 12 mm±10%; and/or wherein a pitch of the elongate reinforcing member is in the range of 1.8 and 3.5 mm±10%.

Preferably the longitudinal distance between corresponding points on adjacent windings of the elongate reinforcing member, measured when the medical tube is not subject to deformational strain is selected such that the elongate film drapes a maximal amount with respect to other selections of the longitudinal distance, between successive windings of the elongate reinforcing member while not extending inwardly beyond the elongate reinforcing member base into the lumen when the medical tube is bent.

Preferably the pitch and the amount of the elongate film extending between adjacent windings of the elongate reinforcing member is selected such that the elongate film extending between adjacent windings of the elongate reinforcing member comprises an arc having an arc length that does not protrude into the lumen of the interface tube.

Preferably the maximal amount of the elongate film which drapes between successive windings of the elongate reinforcing member extends between adjacent windings of the elongate reinforcing member without protruding into the lumen.

Preferably a bead height (BH) of the windings is in the range of 0.8 and 1.5 mm±10%, and/or wherein a bead width (BW) of the windings is in the range of 0.8 and 1.5 mm±10%.

Preferably the elongate film is wrapped around a radially-outward surface of the elongate reinforcing member, facing away from the lumen, such that the elongate reinforcing member interacts with the lumen of the medical tube and the elongate film forms the outer surface of the medical tube.

Preferably windings of the elongate reinforcing member roll or lean sideways in response to an applied force.

Preferably the medical tube maintains a gases flow while the force is applied.

Preferably the longitudinal distance between corresponding points on adjacent windings, measured when the medical tube is not subject to deformational strain, is selected to maintain a bend radius greater than or equal to 10 mm.

Preferably the medical tube is connected to a patient interface, preferably the medical tube can connect between an inspiratory tube and the patient interface.

Preferably the medical tube further comprises a connector to connect between the inspiratory tube and the patient interface.

Preferably the medical tube is used in high flow therapy.

Preferably the patient interface comprises a nasal cannula.

## Claims

1. A medical tube (200) to transport gases, the medical tube (200) comprising:
an elongate film (210) spirally wrapped with an elongate reinforcing member (220) to form a lumen, the elongate reinforcing member (220) comprising a D-shaped cross section, the elongate film bonding to a semi-circular part of the D-shaped cross section of the elongate reinforcing member (220),
wherein a maximal amount of the elongate film (210) extends between adjacent windings of the elongate reinforcing member (220) without protruding, beyond an elongate reinforcing member base, into the lumen.

2. The medical tube (200) as claimed in claim 1, wherein the elongate film (210) is made of a breathable material.

3. The medical tube (200) as claimed in claims 1 or 2, wherein the elongate film (210) is wrapped around a radially-outward surface of the elongate reinforcing member (220), facing away from the lumen, such that the elongate reinforcing member (220) interacts with the lumen of the medical tube (200) and the elongate film (210) forms the outer surface of the medical tube.

4. The medical tube (200) as claimed in any one of claims 1 to 3, wherein the average radial distance from the lowest point of the elongate film (210) in the lumen to the bottom of the elongate reinforcing member (220), measured when the medical tube (200) is not subject to deformational strain, is less than 0.2 mm.

5. The medical tube (200) as claimed in any one of claims 1 to 4, wherein the profile of the elongate film (210) is an inwardly biased profile.

6. The medical tube (200) as claimed in any one of claims 1 to 5, wherein the flat part of the D-shaped cross section is longitudinally aligned with the lumen and the semi-circular part of the D-shaped cross section is facing away from the lumen.

7. The medical tube (200) as claimed in any one of claims 1 to 6, wherein windings of the elongate reinforcing member (220) roll or lean sideways in response to an applied force.

8. The medical tube (200) as claimed in claim 7, wherein the medical tube (200) maintains a gases flow while the force is applied.

9. The medical tube (200) as claimed in any one of claims 1 to 8, wherein the elongate reinforcing member (220) is hollow.

10. The medical tube (200) as claimed in any one of claims 1 to 9, wherein the longitudinal distance between corresponding points on adjacent windings, measured when the medical tube (200) is not subject to deformational strain, is selected to maintain a bend radius greater than or equal to 10 mm.

11. The medical tube (200) as claimed in any one of claims 1 to 10, wherein the elongate film (210) is made of a thermoplastic polyurethane material.

12. The medical tube (200) as claimed in any one of claims 1 to 11, wherein the elongate film (210) is made of a material having a moisture vapour transmission rate greater than or equal to 650 g/m2/day, when measured according to Procedure B of ASTM E96, using the upright cup method at a temperature of 23 °C and a relative humidity of 50%.

13. The medical tube (200) as claimed in in any one of claims 1 to 12, wherein the diameter of the medical tube (200) is between 1 mm and 16 mm.

14. The medical tube (200) as claimed in any one of claims 1 to 13, wherein the longitudinal distance between corresponding points on adjacent windings of the elongate reinforcing member (220), measured when the medical tube (200) is not subject to deformational strain of the elongate reinforcing member (220), is between 2.0 mm and 2.5 mm.

15. The medical tube (200) as claimed in any one of claims 1 to 14, wherein the pitch and the amount of the elongate film (210) extending between adjacent windings of the elongate reinforcing member (220) is selected such that the elongate film (210) extending between adjacent windings of the elongate reinforcing member (220) comprises an arc having an arc length that does not protrude into the lumen of the medical tube (200).
